# EUROPÄISCHE PATENTANMELDUNG

(11) **EP 2 113 241 A2**
(43) Veröffentlichungstag der Anmeldung: **04.11.2009**
(21) Anmeldenummer: 09159078.6
(22) Anmeldetag: 29.04.2009
(51) Int. Cl.: A61K 8/11, A61K 8/14, A61K 8/67, A61K 8/73, A61Q 19/08

(54) **Kosmetisches Erzeugnis mit verzögerter Retinolfreisetzung**

(30) Priorität: 29.04.2008 DE 102008022041
(71) Anmelder: Coty Prestige Lancaster Group GmbH, 55116 Mainz (DE)
(72) Erfinder: Golz-Berner, Karin, 98000, Monaco (MC); Zastrow, Leonhard, 98000, Monaco (MC)
(74) Vertreter: Gulde Hengelhaupt Ziebig & Schneider

(57) **Zusammenfassung**

Die Erfindung betrifft ein kosmetisches Erzeugnis mit verzögerter Retinolfreisetzung, enthaltend lamellenartiges gesättigtes (hydriertes) Phosphatidylcholin, in dessen lamellenartiger Struktur 20.000 bis 100.000 IU Retinol eingelagert sind; Liposome auf Basis von hydrolysiertem Sojaprotein, enthaltend Retinol (1000 bis 200000 IU), Cyclodextrine, enthaltend 80.000 bis 100.000 IU Retinol; ein Transportsystem mit einer Chitosan/Carboxymethylcellulose-Hülle, enthaltend Retinol (3000 bis 200000 IU), und kosmetische Hilfsstoffe, Trägerstoffe oder Gemische davon. Das Produkt zeigt sowohl eine sehr kurzfristig auftretende erhöhte Anti-Faltenwirkung als auch eine langanhaltende signifikant verbesserte Anti-Faltenwirkung mit konstanter Retinolabgabe.

## Beschreibung

Die Erfindung betrifft ein kosmetisches Erzeugnis mit verzögerter Retinolfreisetzung, das eine verbesserte Anti-Alterungswirkung für die Haut zeigt.

Retinol ist seit langem als bevorzugter Wirkstoff für die Haut bekannt und in vielen Kombinationen eingesetzt worden. Problematisch bei Retinol ist dessen Stabilität und damit kurze Einwirkungsmöglichkeit auf die zellulären Hautstrukturen, da es sehr schnell abgebaut wird. Aus diesem Grunde existieren auch zahlreiche Vorschläge, Retinol mit anderen Substanzen wie z.B. Citronensäure, Monoalkylphosphaten, Histidin usw. zu stabilisieren. Stabiler als Retinol selbst sind dessen Fettsäurederivate, deren Schutzwirkung infolge ihrer Stabilität jedoch weitaus geringer ist.

Aus der WO 02/080875 ist eine Kombination verschiedener Retinol-Darreichungsformen bekannt, die durch ein freies Retinylderivat, ein liposomal verkapseltes Retinylderivat, verkapseltes Retinol und ein Pflanzenextrakt(gemisch) eine Steigerung bei der Anti-Faltenwirkung erreicht.

Der Erfindung liegt die Aufgabe zugrunde, die Anti-Faltenwirkung bekannter kosmetischer Produkte weiter zu verbessern.

Erfindungsgemäß bereitgestellt wird daher ein kosmetisches Erzeugnis mit verzögerter Retinolfreisetzung, gekennzeichnet durch einen Gehalt an
0,1 bis 2,5 Gew.-% lamellenartigen gesättigten Phosphatidylcholins, in dessen lamellenartiger Struktur 20.000 bis 100.000 Internationale Einheiten (IU) Retinol eingelagert sind;
0,1 bis 3,0 Gew.-% eines Transportsystems mit einer Chitosan-Carboxylmethylcellulose-Hülle, enthaltend 3.000 bis 200.000 IU Retinol;
0,1 bis 2 Gew.-% Cyclodextrine, enthaltend 80.000 bis 100.000 IU Retinol;
0,1 bis 2 Gew.-% von auf hydrolysiertem Sojaprotein basierenden Liposomen, enthaltend 1.000 bis 200.000 IU Retinol; und
einen restlichen Gehalt bis 100 Gew.-% kosmetische Hilfsstoffe, Trägerstoffe oder Gemische davon.

Phosphatidylcholin ist einer der bekanntesten Vertreter der Phosopholipide und wird üblicherweise durch Extraktion aus Lecithin hergestellt. In Verbindung mit Wasser bildet Phosphatidylcholin spontan doppelschichtige Micellen aus, die einen Hohlraum einschließen. Dies wird seit langem zur Verkapselung von Wirkstoffen in Form von sogenannten Liposomen genutzt. Durch den Aufbau dieser Liposome lassen sie sich leicht in die Barriereschicht der Haut integrieren. Die Haut wird dadurch durchlässiger, und verkapselte Wirkstoffe können innerhalb der Barriereschichten und darüber hinaus abgegeben werden. Allerdings wird durch die Durchbrechung der Barriereschichten auch der transepidermale Wasserverlust vergrößert und der natürliche Schutzeffekt der Haut minimiert. Daher dürfen Liposomen in kosmetischen Produkten nur in geringen Mengen eingesetzt werden bzw. Liposomen-haltige kosmetische Produkte sind für sensible und trockene Haut nur eingeschränkt empfehlenswert.

Die wesentlichen Bestandteile des Phosphatidylcholins sind Linolsäure und Cholin. Wenn die ungesättigte Linolsäure durch gesättigte Palmitinsäure und Stearinsäure ersetzt wird, erhält man ein Produkt (INCl: Hydrogenated Lecithin), das keine micellenförmigen Strukturen bildet, sondern solche, die plattenartig oder lamellenartig angeordnet sind. Dieses lamellenartige gesättigte Phosphatidylcholin restauriert und stabilisiert infolge seiner strukturellen Ähnlichkeit die Barriereschichten der Haut. Mit Liposomen können daher die Barriereschichten der Haut geöffnet und mit dem lamellenartigen System wieder geschlossen werden.

Es wurde gefunden, dass in die lamellenartige Struktur des gesättigten Phosphatidylcholins eingelagertes Retinol sehr langsam aus den Barriereschichten der Haut in die Umgebung abgegeben wird. In Kombination mit weiteren Transportsystemen für Retinol, wie z.B. Chitosan-Carboxymethylcellulosehüllen, auf hydrolysiertem Sojaprotein basierenden Liposomen und Cyclodextrin, wird erfindungsgemäß eine Abgabe von Retinol über einen längeren Zeitraum mit der maximalen Retinolkonzentration von etwa 0,5 Gew.-% erreicht.

Dabei wurden erfindungsgemäß Retinol-Carriersysteme gemischt, die eine schnelle, eine mittelfristige und eine langfristige Freisetzung von Retinol ermöglichen. Somit wird mit der erfindungsgemäßen Mischung eine kosmetische Zusammensetzung bereit gestellt, die kontinuierlich und über einen längeren Zeitraum (von über 8 Monaten) Retinol freisetzt. Diese gleichmäßige Abgabe scheint die Ursache für eine besonders gute Tiefen- und Langzeitwirkung des Erzeugnisses zu sein. Somit wird durch das erfindungsgemäße Produkt sowohl eine sehr kurzfristig auftretende erhöhte Anti-Faltenwirkung als auch eine langanhaltende signifikant verbesserte Anti-Faltenwirkung erreicht.

Es wird bei einmaliger Anwendung eine Langzeitwirkung über 24 Stunden erreicht, die deutlich höher ist als die Summe der einzelnen Komponenten des Erzeugnisses. Somit kann durch die erfindungsgemäße Kombination der Retinol-Transportsysteme eine synergistische Wirkung von Retinol auf die Haut erreicht werden. Eine weitere besonders vorteilhafte Wirkung besteht darin, dass das Erzeugnis auch bei längerer Lagerung (z.B. 6 Monate und länger) seine weiße Farbe beibehält und keine Gelbfärbung auftritt, was üblicherweise bei Vitamin-A-Derivate enthaltenden Kosmetika beobachtet wird.

Eine weitere vorteilhafte Wirkung ist die überraschende Tiefenwirkung, die auch einen gewissen Ausgleich tieferer Hautfalten ermöglicht.

Bevorzugte Bereiche für die aktiven Bestandteile des retinolhaltigen Kosmetikproduktes sind 0,3 bis 1,5 Gew.-% des lamellenartigen gesättigten Phosphatidylcholins, noch bevorzugter 0,5 bis 1 Gew.-%, 0,2 bis 1,5 Gew.-% des Transportsystems Chitosan/Carboxymethylcellulose, noch bevorzugter 0,5 bis 1 Gew.-% und/oder 0,2 bis 1,3 Gew.-% Cyclodextrine, noch bevorzugter 0,5 bis 1 Gew.-% jeweils mit den entsprechenden Anteilen (IU) an Retinol. Ein bevorzugter Bereich für die Liposome, die auf Sojaprotein basieren, liegt bei 0,2 bis 0,9 Gew.-%.

Der Anteil (IU) an eingeschlossenem Retinol liegt bei den lamellenartigen gesättigten Phosphatidyl Trägersystemen vorzugsweise bei 50.000 bis 100.000, noch bevorzugter bei 50.000. Bei den Chitosan/Carboxymethylcellulose-Hüllen liegt ein bevorzugter Anteil (IU) bei 70.000. Bei Cyclodextrin als Trägersystem liegt der Anteil (IU) an eingeschlossenem Retinol vorzugsweise bei 100.000 und/oder bei den auf hydrolysiertem Sojaprotein basierten Liposomen vorzugsweise bei 80.000 bis 100.000, noch bevorzugter bei 80.000 IU.

Ein vorteilhaftes Transportsystem mit einer Hülle aus Chitosan/Carboxymethylcellulose ist z.B. Primashere® L2 von Cognis, Deutschland. Ein vorteilhaftes Cyclodextrin ist z.B. Retinol W8 von Wacker-Chemie, Deutschland, oder von CLR Dr. Kurt Richter, Deutschland. Ein vorteilhaftes Liposom auf Basis von Sojaprotein ist Cytovector® von Engelhard, USA/Deutschland. Als lamellenartiges gesättigtes Phosphatidylcholin wird z.B. das Derma Membran System/Derma Membran Struktur (DMS) von Kuhs GmbH, Deutschland verwendet.

Das erfindungsgemäße kosmetische Produkt enthält neben den genannten Retinol-Wirkstoffen weitere kosmetisch annehmbare Hilfsstoffe oder Trägerstoffe oder Gemische davon. Als kosmetische Hilfsstoffe werden in diesem Zusammenhang alle Begleitstoffe bezeichnet, die gegebenenfalls auch eine eigene Wirkung aufweisen, wie z.B. UV-Filter oder die Bräunung oder Aufhellung der Haut beeinflussende Mittel, Radikalfänger, Enzyme, Wachse, pflanzliche Wirkstoffe, Polymere, Melanin, Antioxidationsmittel, entzündungshemmende natürliche Wirkstoffe, Ubichinon Q10, Kreatin, Kreatinin, Camitin, Biotin, Isoflavone, Isoflavonoide, Cardiolipin, Liponsäure, Hautfunktionen verbessernde Wirkstoffe wie z.B. Grüntee-Extrakte, Eucalyptusöl, Harnstoff, Mineralsalze, Meeresmineralien und/oder Osmolyte, Feuchthaltemittel, Weichmacher oder Gemische davon.

Weitere Hilfsstoffe sind Konservierungsmittel, Farbstoffe, Pigmente mit färbender Wirkung, Füllstoffe wie z.B. SiO₂ oder Silica, Verdickungsmittel, Duftstoffe, Alkohole, Polyole, Ester, Elektrolyte, Gelbildner, Filmbildner, Polymere, Copolymere, Wachse, Stabilisatoren, Emulgatoren oder Gemische davon.

Zu Antioxidationsmitteln gehören Vitamine wie z.B. Vitamin C und Derivate davon, beispielsweise Ascorbylacetat, -phosphat und -palmitat, Magnesiumascorbylphosphat; Folsäure und deren Derivate; Vitamin E und deren Derivate, wie z.B. Tocopherylacetat; Flavone oder Flavonoide; Aminosäuren, wie z.B. Histidin, Glycin, Tyrosin, Tryptophan und Derivate davon; Imidazole wie z.B. cis- oder trans-Urocaninsäure und deren Derivate; Peptide wie z.B. D,L-Carnosin, D-Carnosin, L-Carnosin und deren Derivate; Carotinoide und Carotine, wie z.B. α-Carotin, β-Carotin, Lycopin; Harnsäure und Derivate davon; α-Hydroxysäuren wie z.B. Citronensäure, Milchsäure, Apfelsäure; α-Hydroxyfettsäuren wie z.B. Palmitinsäure, Phytinsäure, Lactoferrin; Stilbene und deren Derivate; Mannose und deren Derivate; Ferulasäure und deren Derivate; Thiole wie z.B. Glutathion, Cystein, Cystin und deren Ester.

Erfindungsgemäß ausgeschlossen aus der Gruppe der verwendeten kosmetischen Hilfs- und Trägerstoffe sind die Extrakte der Pflanze *Passiflora incarnata* sowie deren aktive Wirkstoffe umfassend Flavone vom Typ Apigenin, Luteolin oder deren C-Glycoside sowie Vitex und Isovitex als auch die Extrakte der Pflanze *Vaccinium myrtillus* sowie deren aktive Wirkstoffe umfassend Anthocyanoside vom Typ Delphinidin. Diese Substanzen werden erfindungsgemäß daher auch nicht als Antioxidationsmittel verwendet.

Ein bevorzugtes Antioxidationsmittel ist ein Gemisch mit einem Gehalt an einem durch Extraktion der Rinde von Quebracho blanco und nachfolgender enzymatischer Hydrolyse gewonnenem Produkt, das wenigstens 90 Gew.-% Proanthocyanidin-Oligomere und höchstens 10 Gew.-% Gallussäure enthält, in Mikrokapseln, sowie mit einem durch Extraktion gewonnenen Seidenraupen-extrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser, gemäß WO 99/66881 (Anspruch 1) (RPF-Komplex 1).

Ein weiteres bevorzugtes Antioxidationsmittel ist ein Gemisch aus Angelica archangelica-Wurzelextrakt (INCl: Angelica archangelica root extract, CAS number 84775-41-7), Pongamia pinnata-Samenextrakt (INCl: Pongamia pinnata seed extract), Camelia sinensis-Blattextrakt (INCl: Camellia sinensis Leaf Extract, CAS number 84650-60-2), Coffea arabica-Samenextrakt (INCl: Coffea arabica (coffee) seed extract, CAS number 84650-00-0), Ethanol im Verhältnis 0,2 : 0,2 : 0,2 : 0,2 : 99,2 gemäß W02004/105706 (Anspruch 2) (RPF-Komplex 2).

Ein besonders bevorzugtes Antioxidationsmittel ist eine Wirkstoffzubereitung, die ein in Lecithin verkapseltes Gemisch aus (in Gew.-%) 2 % Angelica archangelica-Wurzelextrakt 2 % Pongamia pinnata-Samenextrakt, 2 % Camelia sinensis-Blattextrakt und 2 % Coffea arabica-Samenextrakt beinhaltet, wobei außerdem 8 % Glycerin, 8,25 % Alkohol denaturiert, Antioxidationsmittel und Hilfsstoffe enthalten sind (RPF-Komplex classic).

Als Wachse können natürliche pflanzliche Wachse, tierische Wachse, natürliche und synthetische Mineralwachse und synthetische Wachse sowie Wachsgemische verwendet werden. Zu den bevorzugten Wachsen gehören beispielsweise Carnaubawachs, Candellilawachs, Bienenwachs, Wollwachs, Hartparaffin, Ceresin, Ozokerit, Siliconwachs, Polyethylenglycolwachse oder Polyethylenglycolesterwachse.

Als Trägerstoffe können eingesetzt werden Wasser, polare und unpolare Öle, nicht oder wenig färbende Pigmente, Pulver oder Mischungen davon.

Zu Pigmenten für das erfindungsgemäße Produkt gehören Titandioxid, Zinkoxid, Aluminiumoxid, Siliciumdioxid, Silicate, Glimmer, Kaolin, Calciumcarbonat, Talkum, Nylonkügelchen, Keramikkügelchen, expandierte und nichtexpandierte synthetische Polymerpulver, pulverförmige natürliche organische Verbindungen wie z.B. gemahlene Festalgen, gemahlene Pflanzenteile, verkapselte und unverkapselte Getreidestärken oder Gemische davon.

Besonders vorteilhaft ist der Einsatz von farblosem Quarzpulver als Mineralkörper mit Teilchengrößen von 2 - 20 µm. Damit wird eine zusätzliche Anti-Faltenwirkung erreicht, wobei ein Auffülleffekt tieferer Hautfalten zu beobachten ist.

Die für die Erfindung eingesetzten Öle können übliche kosmetische Öle oder kosmetische Ester oder Ether oder Gemische davon sein. Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycoldiheptanoat, PPG-15-stearylether sowie pflanzliche Öle, wie z.B. Calendulaöl, Jojobaöl, Avocadoöl, Macadamianußöl, Rizinusöl, Kakaobutter, Kokosnußöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Das erfindungsgemäße Kosmetikprodukt ist vorzugsweise ein Produkt der dekorativen Kosmetik, ein Körperpflegeprodukt und/oder ein Sonnenschutzprodukt. Die Verwendung des erfindungsgemäßen kosmetischen Produktes erfolgt somit vorzugsweise z.B. in Make-ups, Foundations, Sonnencremes, Sonnengelen, After-sun-Produkten, Tagescremes, Nachtcremes, Masken, Körperlotionen, Augenkosmetika.

Erfindungsgemäß können die retinolhaltigen Trägersysteme mit einem oder mehreren Hilfsstoffen und/oder Trägerstoffen kombiniert werden. In einer bevorzugten Ausgestaltung der Erfindung enthält das kosmetische Produkt lamellenartiges gesättigtes Phosphatidylcholin, in dessen lamellenartiger Struktur 20.000 bis 100.000 Internationale Einheiten (IU) Retinol eingelagert sind, ein 3.000 bis 200.000 IU Retinol enthaltendes Transportsystem mit einer Chitosan/Carboxymethylcellulose-Hülle, 80.000 bis 100.000 IU Retinol enthaltende Cyclodextrine, auf hydrolysiertem Sojaprotein basierende Liposomen enthaltend 1.000 bis 20.000 IU Retinol, ein Antioxidationsmittel, Quarzpulver, ein Feuchthaltemittel und wahlweise Hautkonditionierungsmittel, Weichmacher, Filmbildner, Lösungsmittel, Emulgatoren, Farbstoffe, Füllstoffe, Parfümstoffe und/oder Konservierungsmittel.

Die Herstellung derartiger Produkte erfolgt auf eine Weise, wie sie dem Fachmann auf diesem Gebiet bekannt ist.

Ein weiterer Gegenstand der Erfindung ist die Verwendung des erfindungsgemäßen Kosmetikproduktes zur kosmetischen Anti-Faltenbehandlung der menschlichen Haut.

Gegenstand der Erfindung ist somit auch die Verwendung eines Gemisches aus 0,1 bis 2,5 Gew.-% lamellenartigen gesättigten Phosphatidylcholins, in dessen lamellenartiger Struktur 20.000 bis 100.000 Internationale Einheiten (IU) Retinol eingelagert sind;
0,1 bis 3,0 Gew.-% eines Transportsystems mit einer Chitosan/Carboxymethylcellulose-Hülle, enthaltend 3.000 bis 200.000 IU Retinol;
0,1 bis 2,0 Gew.-% Cyclodextrine, enthaltend 80.000 bis 100.000 IU Retinol;
0,1 bis 2,0 Gew.-% von auf hydrolysiertem Sojaprotein basierenden Liposomen enthaltend 1.000 bis 20.000 IU Retinol; und
einen restlichen Gehalt bis 100 Gew.-% an kosmetischen Hilfsstoffen, Trägerstoffen oder Gemischen davon zur Anti-Faltenbehandlung der menschlichen Haut.

Erfindungsgemäße Effekte treten bereits bei einmaliger Anwendung des Produkts auf. Bevorzugt erfolgt die kosmetische Behandlung der Haut mit einer Auftragsmenge von 1-5 mg/cm² Haut im Bereich der Gesichtshaut zweimal täglich über einen Zeitraum von 1 bis 10 Tagen, vorzugsweise von 3 bis 10 Tagen. Noch bevorzugter erfolgt die Behandlung über den gleichen Zeitraum einmal täglich.

Weiter bevorzugt erfolgt die Behandlung mit einer Auftragsmenge von 1-5 mg/cm² Haut im Bereich der Gesichtshaut einmalig.

Die Erfindung soll nachstehend durch Beispiele näher erläutert werden. Alle Angaben erfolgen in Gewichtsprozent, sofern nichts anderes angegeben ist.

**Beispiel 1 Anti-Falten Tages- und Nachtcreme I**

| **Phase 1** | |
|---|---|
| Wasser | q.s. ad 100 |
| Glycerine | 3,0 |
| Cyclodextrin & Retinol (100.000 IU) | 0,5 |
| Dimethicone & Cyclotetrasiloxane | 3,0 |
| Cyclopentasiloxane & Dimethiconol & Cyclotetrasiloxane | 6,0 |
| Methylmethacrylate Crosspolymer | 2,0 |
| Butylenglycol | 3,0 |
| Xanthan Gum | 0,1 |

| **Phase 2** | |
|---|---|
| Dicaprylyl Carbonate & Tocopherol | 4,0 |
| Beheneth-10 | 3,0 |
| Behenyl Alcohol | 1,0 |
| Shea Butter | 4,0 |
| Cyclopentasiloxane & Cyclohexasiloxane | 3,0 |
| Hydrogenated Polyisobutene & Tocopherol | 4,0 |

| **Phase 3** | |
|---|---|
| Polyacrylamide & Water & C13-14 Isoparaffin & Laureth-7 | 2,0 |
| Retinol in Liposomen (70.000 IU) Chitosan/Carboxymethylcellulose | 0,5 |
| Sojaprotein-Liposomen & Retinol (80.000 IU) | 0,5 |
| DMS Retinol (Kuhs GmbH, Deutschland)*¹ (50.000 IU) | 0,5 |

| **Phase 4** | |
|---|---|
| Konservierungsmittel | 0,6 |
| Parfüm | 0,7 |
| Mica & Silica & Titanium Dioxide | 0,6 |
| Caprylyl Glycol & 1,2-Hexanediol & Tropolone | 0,5 |
| Quartz & Potassium Sorbate & Sorbic Acid & Phosphoric Acid | 0,1 |
| Imperata Root Extract | 1,0 |

| | |
|---|---|
| *¹ Retinol & Hydrogenated Lecithin & Caprylic/Capric Triglyceride & Shea Butter & Squalane. | |

Die Phasen 1 und 2 wurden separat bei etwa 70°C hergestellt und dann unter Rühren zusammengeführt. Nach dem Abkühlen auf etwa 40°C wurde die Phase 3 hinzugegeben. Danach erfolgte die Zugabe der Phase 4 bei etwa 30 °C.

Es wurde eine sehr helle Creme ohne Gelbton erhalten.

### Beispiel 2 Anti-Falten Tages- und Nachtcreme II

Die Zusammensetzung gemäß Beispiel 1 wurde in der Phase 4 durch 0,5 % RPF-Komplex I ergänzt, bestehend aus Wasser, 1 % Gelbildner, 7,5 % Phospholipide, 2 % Quebracho-Extrakt und 1 % Seidenraupen-Extrakt (gemäß WO 99/66881, Anspruch 1), bezogen auf das Gesamtgewicht des Komplexes.

### Beispiel 3 Anti-Falten Tages- und Nachtcreme III

Die Zusammensetzung gemäß Beispiel 1 wurde in der Phase 4 durch 0,5 % RPF-Komplex classic ergänzt, bestehend aus 3,5 % Lecithin, 2 % Angelica archangelica root extract, 2 % Pongamia pinnata seed extract, 2 % Camelia sinensis Leaf Extract, 2 % Coffea arabica seed extract, 8 % Glycerin, 8,25 % Alkohol denaturiert, 0,3 % Zitronensäure, 0,2 % Ascorbyl Palmitate, 0,2 % Tocopherol, 0,2 % Ascorbinsäure, 1,0 % Guar Hydroxypropyltrimonium chlorid, 0,2 % PEG-8, 0,28 % Konservierungsmittel und q. s. ad 100 Wasser, wobei der RPF-Komplex classic in Lecithin verkapselt vorliegt. Die Prozentangaben sind Gewichtsprozent, die auf das Gesamtgewicht des Komplexes bezogen sind.

**Beispiel 4 Anti-Falten Nachtcreme**

| **Phase 1** | |
|---|---|
| Wasser | q.s. ad 100 |
| Glycerine | 3,0 |
| Cyclodextrin & Retinol (100.000 IU) | 0,5 |
| Dimethicone & Cyclotetrasiloxane | 3,0 |
| Cyclopentasiloxane & Dimethiconol & Cyclotetrasiloxane | 6,0 |
| Methylmethacrylate Crosspolymer | 2,0 |
| Butylenglycol | 3,0 |
| Xanthan Gum | 0,1 |

| **Phase 2** | |
|---|---|
| Dicaprylyl Carbonate & Tocopherol | 4,0 |
| Beheneth-10 | 3,0 |
| Behenyl Alcohol | 1,0 |
| Shea Butter | 4,0 |
| Cyclopentasiloxane & Cyclohexasiloxane | 3,0 |
| Hydrogenated Polyisobutene & Tocopherol | 4,0 |

| **Phase 3** | |
|---|---|
| Retinol in Liposomen (70.000 IU) (Retinol Primasphere L2) | 0,7 |
| Sojaprotein-Liposome & Retinol (100.000 IU) | 2,4 |
| DMS Retinol (Kuhs GmbH, Deutschland) (100.000 IU) | 0,7 |

| **Phase 4** | |
|---|---|
| Konservierungsmittel | 0,6 |
| Parfüm | 0,7 |
| Mica & Silica & Titanium Dioxide | 0,6 |
| Caprylyl Glycol & 1,2-Hexanediol & Tropolone | 0,5 |
| Quartz & Potassium Sorbate & Sorbic Acid & Phosphoric Acid | 0,1 |
| Imperata Root Extract | 1,0 |

| | |
|---|---|
| *¹ Retinol & Hydrogenated Lecithin & Caprylic/Capric Triglyceride & Shea Butter & Squalane. | |

Es wurde eine sehr helle Creme ohne Gelbton erhalten.

**Beispiel 5 Anti-Falten Nachtcreme II**

| **Phase 1** | |
|---|---|
| Wasser | q.s. ad 100 |
| Glycerine | 3,0 |
| Cyclodextrin & Retinol (100.000 IU) | 0,5 |
| Dimethicone & Cyclotetrasiloxane | 3,0 |
| Cyclopentasiloxane & Dimethiconol & Cyclotetrasiloxane | 6,0 |
| Methylmethacrylate Crosspolymer | 2,0 |
| Butylenglycol | 3,0 |
| Xanthan Gum | 0,1 |

| **Phase 2** | |
|---|---|
| Dicaprylyl Carbonate & Tocopherol | 4,0 |
| Beheneth-10 | 3,0 |
| Behenyl Alcohol | 1,0 |
| Shea Butter | 4,0 |
| Cyclopentasiloxane & Cyclohexasiloxane | 3,0 |
| Hydrogenated Polyisobutene & Tocopherol | 4,0 |

| **Phase 3** | |
|---|---|
| Retinol in Liposomen (70.000 IU) (Retinol Primasphere L2) | 0,7 |
| Sojaprotein-Liposome & Retinol (100.000 IU) | 2,0 |
| DMS Retinol (Kuhs GmbH, Deutschland) (100.000 IU) | 0,7 |

| **Phase 4** | |
|---|---|
| Konservierungsmittel | 0,6 |
| Parfüm | 0,7 |
| Mica & Silica & Titanium Dioxide | 0,6 |
| Caprylyl Glycol & 1,2-Hexanediol & Tropolone | 0,5 |
| Quartz & Potassium Sorbate & Sorbic Acid & Phosphoric Acid | 0,1 |
| Imperata Root Extract | 1,0 |

| | |
|---|---|
| *¹ Retinol & Hydrogenated Lecithin & Caprylic/Capric Triglyceride & Shea Butter & Squalane. | |

Es wurde eine sehr helle Creme ohne Gelbton erhalten.

### Beispiel 6 (Vergleichsbeispiel 1) Retinolfreisetzung

Es erfolgte eine Überprüfung der Retinolfreisetzung einer erfindungsgemäßen Creme. Die Messung der Proben (3 Parallelproben) für die Creme nach Beispiel 1 erfolgte in Abständen von 1 - 2 Monaten über einen Zeitraum von 8 Monaten.

Die Messung mittels HPLC erfasste α- und γ-Tocopherol sowie all-trans-Retinol. Es wurde gefunden, dass die Konzentration von Gesamt-Retinol bei der fünfmaligen Messung, beginnend am Tag der Cremeherstellung bis zum Ende des 8. Monats danach stets im Bereich von 0,42 bis 0,54 Gew.-% lag, bezogen auf das Gesamtgewicht der Creme. Dies zeigt die erwünschte Freisetzung des Retinols mit einer nahezu gleichmäßigen und wirksamen Konzentration durch das Zusammenwirken der Einzelkomponenten des kosmetischen Erzeugnisses.

### Beispiel 7 (Vergleichsbeispiel 2) Anwender-Test

Ein Anwendertest mit 30 Teilnehmerinnen im Alter von 33 bis 49 Jahren wurde durchgeführt. Alle Teilnehmerinnen trugen einmal täglich (morgens) eine Creme mit etwa 2 mg/cm² im Gesicht auf, insbesondere um Augen- und Mundpartie des Gesichts. Die Behandlung wurde nach 10 Tagen beendet.

Vom Ausgangszustand und nach 12 Stunden, 24 Stunden, 2 Tagen, 4 Tagen, 8 Tagen und 10 Tagen wurden digitalisierte Aufnahmen vom Augenwinkel und einer weiteren ausgewählten Gesichtspartie der Teilnehmerinnen gemacht und über ein Computerprogramm nach Anzahl der Falten und Faltentiefe ausgewertet.

Die Creme hatte folgende Zusammensetzung:

### Phase 1

Wasser q.s ad 100; Glycerine 5,0 %; Butylenglycole 4,0 %; Xanthan Gum 0,5 %

### Phase 2

Dicaprylyl Carbonate 3,0 %; Beheneth-10 3,0 %; Dimethicone 6,0 %; Shea Butter 3,5 %; Cyclodextrin & Retinol 0,5 %

### Phase 3

Silicone DC 200 3,0 %; Konservierungsmittel 0,5 %; Parfüm 0,5 %; und zusätzlich in Phase 3
Gruppe 1: Cyclodextrin & Retinol (100.000 IU) 0,5 %
Gruppe 2: Retinol (70.000 IU) in Liposomen mit einer Hülle aus Chitosan/Carboxymethylcellulose 0,4 %
Gruppe 3: Sojaprotein-Liposomen & Retinol (80.000 IU) 0,5 %
Gruppe 4: DMS Retinol (Kuhs GmbH, Deutschland) (50.000 IU) 0,5 %
Gruppe 5: Retinol (70.000 IU) in Liposomen aus Chitosan/Carboxymethylcellulose 0,2 %; Cyclodextrin & Retinol (100.000 IU) 0,5 %; DMS Retinol (50.000 IU) (Kuhs GmbH, Deutschland) 0,5 %, Sojaprotein-Liposomen (80.000 IU) 0,5 %.

Die Ergebnisse dieses Anwendungstests sind in Tabelle 1 dargestellt.

**Tabelle 1**

| **Faltenreduzierung [%]** | | | | | |
|---|---|---|---|---|---|
| | **Gruppe** | | | | |
| | **1** | **2** | **3** | **4** | **5** |
| Start | - | - | - | - | - |
| nach 12 Std. | 3 | 2 | 2 | 2 | 14 |
| nach 24 Std. | 4 | 3 | 2 | 2 | 21 |
| nach 2 Tagen | 4 | 4 | 2,5 | 4 | 24 |
| nach 4 Tagen | 5 | 4,5 | 4 | 5 | 29 |
| nach 8 Tagen | 5 | 5 | 4 | 7 | 32 |
| nach 10 Tagen | 6 | 5 | 5 | 8 | 39 |

Tabelle 1 zeigt deutlich die überraschend hohe Wirksamkeit der Creme mit der erfindungsgemäßen Kombination gegenüber einer alleinigen Anwendung der Einzelkomponenten. Insbesondere die schnelle Wirkung schon nach 12 und 24 Stunden und die noch immer hohe Langzeitwirkung bei fortgesetzter Anwendung bis zu 10 Tagen sind herausragend.

## Patentansprüche

1. Kosmetisches Erzeugnis mit verzögerter Retinolfreisetzung, **gekennzeichnet durch** einen Gehalt an
0,1 bis 2,5 Gew.-% lamellenartigen gesättigten Phosphatidylcholins, in dessen lamellenartiger Struktur 20.000 bis 100.000 Internationale Einheiten (IU) Retinol eingelagert sind;
0,1 bis 3,0 Gew.-% eines Transportsystems mit einer Chitosan/Carboxymethylcellulose-Hülle, enthaltend 3.000 bis 200.000 IU Retinol;
0,1 bis 2,0 Gew.-% Cyclodextrine, enthaltend 80.000 bis 100.000 IU Retinol;
0,1 bis 2,0 Gew.-% von auf hydrolysiertem Sojaprotein basierenden Liposomen enthaltend 1.000 bis 200.000 IU Retinol; und
einen restlichen Gehalt bis 100 Gew.-% an kosmetischen Hilfsstoffen, Trägerstoffen oder Gemischen davon.

2. Kosmetikprodukt nach Anspruch 1, **dadurch gekennzeichnet, dass** es 0,3 bis 1,5 Gew.-% lamellenartiges gesättigtes Phosphatidylcholin enthält.

3. Kosmetikprodukt nach Anspruch 1 oder 2, **dadurch gekennzeichnet, dass** es 0,2 bis 1,5 Gew.-% Liposome auf Basis des Transportsystems Chitosan/Carboxymethylcellulose enthält.

4. Kosmetikprodukt nach einem der Ansprüche 1 bis 3, **dadurch gekennzeichnet, dass** es 0,2 bis 1,3 Gew.-% Cyclodextrine mit Retinol enthält.

5. Kosmetikprodukt nach einem der Ansprüche 1 bis 4, **dadurch gekennzeichnet, dass** es 0,2 bis 0,9 Gew.-% Liposome, die auf Sojaprotein basieren, enthält.

6. Kosmetikprodukt nach einem der Ansprüche 1 bis 5, **dadurch gekennzeichnet, dass** es als Hilfsstoff ein Antioxidationsmittel enthält, bestehend aus einem Gemisch mit einem Gehalt an einem durch Extraktion der Rinde von Quebracho blanco und nachfolgender enzymatischer Hydrolyse gewonnenem Produkt, das wenigstens 90 Gew.-% Proanthocyanidin-Oligomere und höchstens 10 Gew.-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

7. Kosmetikprodukt nach einem der Ansprüche 1 bis 6, **dadurch gekennzeichnet, dass** es als Hilfsstoff ein Antioxidationsmittel enthält, das ein in Lecithin verkapseltes Gemisch aus 2 Gew.-% Angelica archangelica root extract, 2 Gew.-% Pongamia pinnata seed extract, 2 Gew.-% Camellia sinensis Leaf Extract, 2 Gew.-% Coffea arabica (coffee) seed extract, 8 Gew.-% Glycerin und 8,25 Gew.-% Ethanol denaturiert umfasst.

8. Kosmetikprodukt nach einem der Ansprüche 1 bis 7, **dadurch gekennzeichnet, dass** es als kosmetischen Hilfsstoff mineralischen farblosen Quarz enthält.

9. Kosmetikprodukt nach einem der Ansprüche 1 bis 8, **dadurch gekennzeichnet, dass** es ein Produkt der dekorativen Kosmetik, ein Körperpflegeprodukt und/oder ein Sonnenschutzprodukt ist.

10. Kosmetikprodukt nach Anspruch 9, **dadurch gekennzeichnet, dass** das Produkt ein Make-up, eine Foundation, ein Augenkosmetikum, eine Maske, eine Körperlotion, eine Tagescreme, eine Nachtcreme, eine Sonnencreme, ein Sonnengel und/oder ein After-sun-Produkt ist.

11. Verwendung eines Gemisches aus 0,1 bis 2,5 Gew.-% lamellenartigen gesättigten Phosphatidylcholins, in dessen lamellenartiger Struktur 20.000 bis 100.000 Internationale Einheiten (IU) Retinol eingelagert sind;
0,1 bis 3,0 Gew.-% eines Transportsystems mit einer Chitosan/Carboxymethylcellulose-Hülle, enthaltend 3.000 bis 200.000 IU Retinol;
0,1 bis 2,0 Gew.-% Cyclodextrine, enthaltend 80.000 bis 100.000 IU Retinol;
0,1 bis 2,0 Gew.-% von auf hydrolysiertem Sojaprotein basierenden Liposomen enthaltend 1.000 bis 200.000 IU Retinol; und einen restlichen Gehalt bis 100 Gew.-% an kosmetischen Hilfsstoffen, Trägerstoffen oder Gemischen davon zur kosmetischen Anti-Faltenbehandlung der menschlichen Haut.

12. Verwendung eines Produktes nach einem der Ansprüche 1 bis 10 zur kosmetischen Anti-Faltenbehandlung der menschlichen Haut.

13. Verwendung nach Anspruch 11 oder 12, **dadurch gekennzeichnet, dass** die kosmetische Behandlung mit einer Auftragsmenge von 1-5 mg/cm² Haut im Bereich der Gesichtshaut einmal täglich über einen Zeitraum von 1 bis 10 Tagen erfolgt.

14. Verwendung nach Anspruch 11 oder 12, **dadurch gekennzeichnet, dass** die kosmetische Behandlung mit einer Auftragsmenge von 1-5 mg/cm² Haut im Bereich der Gesichtshaut einmalig erfolgt.
